# EUROPÄISCHE PATENTSCHRIFT

(11) **EP 3 581 196 B1**
(45) Veröffentlichungstag und Bekanntmachung des Hinweises auf die Patenterteilung: **26.01.2022**
(21) Anmeldenummer: 18177441.5
(22) Anmeldetag: 13.06.2018
(51) Int. Cl.: A61K 38/17, A61K 39/00, A61P 7/06, A61P 19/10

(54) **INHIBITOREN DES TRANSFERRINREZEPTOR-2 ZUR VERWENDUNG BEI DER BEHANDLUNG VON OSTEOPOROSE**
INHIBITORS OF THE TRANSFERRIN RECEPTOR-2 FOR USE IN THE TREATMENT OF OSTEOPOROSIS
INHIBITEURS DU RÉCEPTEUR 2 DE LA TRANSFERRINE DESTINÉS À ÊTRE UTILISÉS DANS LE TRAITEMENT DE L'OSTÉOPOROSE

(43) Veröffentlichungstag der Anmeldung: 18.12.2019
(73) Patentinhaber: Kymab Limited, Cambridge CB22 3AT (GB)
(72) Erfinder: RAUNER, Dr. Martina, 01309 Dresden (DE); HOFBAUER, Prof. Dr. Lorenz C., 01309 Dresden (DE); PLATZBECKER, Prof. Dr. Uwe, 01326 Dresden (DE); Baschant, Dr. Ulrike, 01277 Dresden (DE)
(74) Vertreter: Zwicker, Jörk

(56) Entgegenhaltungen:
- ANTONELLA NAI ET AL: "The second transferrin receptor regulates red blood cell production in mice", BLOOD, Bd. 125, Nr. 7, 12. Februar 2015 (2015-02-12), Seiten 1170-1179, XP055517820, DOI: 10.1182/blood-2014-
- ALESSIA CALZOLARI ET AL: "Transferrin receptor 2 is frequently and highly expressed in glioblastomas", TRANSLATIONAL ONCOLOGY, Bd. 3, Nr. 2, 1. April 2010 (2010-04-01), Seiten 123-134, XP055518066, DOI: 10.1539/tlo.09274
- TOSHIFUMI FUJIWARA ET AL: "SU0262 - Iron Homeostasis is Critical for Osteoclast Differentiation", AMERICAN SOCIETY FOR BONE AND MINERAL RESEARCH - ASBMR 2014, Bd. 29(Sup.1), 1. Februar 2014 (2014-02-01), Seite S285, XP055518183,
- "Abstracts of the ECTS congress 2017 ED - Fielding Roger A; Reid Kieran F", CALCIFIED TISSUE INTERNATIONAL, NEW YORK, NY, US, Bd. 100, Nr. 1, 11. Mai 2017 (2017-05-11), Seiten 1-174, XP036233897, ISSN: 0171-967X, DOI: 10.1007/S00223-017-0267-2 [gefunden am 2017-05-11]

## Beschreibung

Die Erfindung betrifft Inhibitoren des Transferrinrezeptor-2 zur Verwendung bei der Behandlung von Erkrankungen des Knochens.

Osteoporose ist die häufigste Erkrankung des Knochens, bei der die Knochendichte und die Knochenqualität abnimmt was mit einem erhöhten Frakturrisiko einhergeht. Der Knochenverlust kann verschiedene Ursachen haben und ist auf eine Steigerung der Knochenresorption durch Osteoklasten und eine Hemmung der Knochenneubildung durch Osteoblasten zurückzuführen. Bisherige Therapieformen wie zum Beispiel Bisphosphonate oder monoklonale Antikörper wie Denosumab (Antikörper gegen RANKL) zielen auf eine Hemmung der Osteoklasten und damit der Knochenresorption. Knochenaufbauende, osteoanabole Therapiemöglichkeiten, die gezielt den Knochenaufbau fördern, gibt es bisher nur in Form von Teriparatid, welches nur bei schwerwiegenden Osteoporosen eingesetzt werden darf.

Anämie oder auch Blutarmut ist charakterisiert durch eine zu geringe Menge am sauerstofftragenden Hämoglobin im Blut und kann entweder angeboren oder erworben sein. Die häufigste Form der Anämie ist die Eisenmangelanämie, meistens verursacht durch Mangelernährung oder Blutungen. Die Behandlung von Anämie erfolgt meist ursächlich, so wird bei einer Eisenmangelanämie Eisen medikamentös verabreicht.

Toshifumi Fujiwara et al., "SU0262 - Iron Homeostasis is Critical for Osteoclast Differentiation", American Society for Bone and Mineral Research - ASBMR 2014, offenbart, dass Eisen essentiell für die Differenzierung von Osteoklasten ist und Eisenüberladung bei einer Vielzahl von hämatologischen Erkrankungen mit einer übermäßigen Knochenresorption verbunden ist, sowie dass die Transferrin-abhängige Eisenaufnahme eine wesentliche Rolle bei der Eisenhomöostase bei Osteoklasten spielt.

Baschant et al., "Abstracts of the ECTS congress 2017", Calcif Tissue Int 100, S43 (2017) offenbart, dass Eisenüberladung zur Entwicklung von Osteoporose führen kann und der Transferrinrezeptor 2 (Tfr2) ein kritischer Regulator der Eisenhomöostase ist, da Mutationen im Tfr2-Gen bei Menschen und Mäusen zu Eisenüberladung führen.

Gegenstand der Erfindung ist ein Inhibitor des Transferrinrezeptor-2 zur Verwendung bei der Behandlung von Osteoporose, wobei der Inhibitor ausgewählt ist aus einem Antikörper oder einem Fragment davon, RNA-Interferenz, CRISPR-Cas und einer Rekombinase.

Der Transferrinrezeptor-2 (Tfr2) wird vor allem in der Leber exprimiert, wo er den Eisenstoffwechsel reguliert. Der Verlust von Tfr2 führt in Mäusen und Menschen zu einer Eisenüberladung über eine Herunterregulierung der Expression von Hepcidin in der Leber, die dann zu einer vermehrten Eisenresorption führt. Zusätzlich ist Tfr2 für die Differenzierung von Erythrozyten notwendig.

Ausführungsformen der Erfindung betreffen einen Inhibitor zur Verwendung bei der Behandlung von primären und sekundären Osteoporosen.

Ebenfalls offenbart, aber nicht gemäß der beanspruchten Erfindung, ist ein Inhibitor zur Verwendung bei der Behandlung der tumor-bedingten Anämie bzw. der Anämie im Rahmen chronischer Erkrankungen (Entzündung, Dialyse, Diabetes etc.). In dieser Situation kommt es einer Hochregulierung von Hepcidin, welches via Degradation von Ferroportin zu einer Eisenretention in den Monozyten und Makrophagen führt. Eine lösliche Form des TFR2 würde als Ligandenfalle (BMPs) agieren und damit gleichsam die TFR2 Signalkaskade hemmen, was zu einer Herunterregulierung der Expression von Hepcidin in der Leber und dann zu einer vermehrten Eisenresorption bzw. zur besseren Bereitstellung aus dem RHS führt.

Ebenfalls offenbart, aber nicht gemäß der beanspruchten Erfindung, ist ein Inhibitor zur Verwendung bei der Behandlung von myelodysplastischen Syndromen, beta-Thalassämie, Niereninsuffizienz, und der Anämie of chronic disease (ACD).

In Ausführungsformen der Erfindung interagiert der Inhibitor mit zumindest einer Isoform ausgewählt aus Tfr2α oder Tfr2β.

Offenbart, aber nicht gemäß der beanspruchten Erfindung, ist ein Inhibitor ausgewählt aus einem Peptid, einem Fusionsprotein und einem Aptamer.

Erfindungsgemäß ist der Inhibitor ausgewählt aus einem Antikörper oder Fragment davon, RNA-Interferenz, beispielsweise einer siRNA, shRNA oder einer miRNA, CRISPR-Cas und einer Rekombinase.

Nachfolgend sind unabhängig vom Gegenstand der Erfindung Beispiele für Inhibitoren wiedergegeben:

### Aptamer:

Pegaptanib, ein pegyliertes Aptamer gegen VEGF (Vascular endothelial growth factor) Adamis AP, Altaweel M, Bressler NM, et al. Changes in retinal neovascularization after pegaptanib (Macugen) therapy in diabetic individuals. Ophthalmology 2006; 113(1):23-8.

Aptamer gegen PDGF (Platelet derived growth factor) in der Tumortherapie: Pietras K, Rubin K, Sjoblom T. Inhibition of PDGF receptor signaling in tumor stroma enhances antitumor effect of chemotherapy. Cancer Res 2002;62:5476-84.

### Peptid:

STI-571: Inhibitor von Protein-Tyrosinkinasen (c-Abl, Bcr-Abl, c-kit): Biochem Biophys Res Commun. 2003 Oct 3;309(4):709-17. STI-571: an anticancer protein-tyrosine kinase inhibitor. Roskoski R Jr.

### RNA-Interferenz:

siRNA gegen IL-13 zur Behandlung von Asthma: Lively, TN, Kossen, K, Balhorn, A, Koya, T, Zinnen, S, Takeda, K et al. (2008). Effect of chemically modified IL-13 short interfering RNA on development of airway hyperresponsiveness in mice. J Allergy Clin Immunol 121: 88-94.

siRNA gegen Hsp27 zur Behandlung von pharmako-resistentem Prostata-Krebs: Liu, C, Liu, X, Rocchi, P, Qu, F, lovanna, JL and Peng, L (2014). Arginine-terminated generation 4 PAMAM dendrimer as an effective nanovector for functional siRNA delivery in vitro and in vivo. Bioconjug Chem 25: 521-532.

### Rekombinasen:

Karpinski J, Hauber I, Chemnitz J, Schäfer C, Paszkowski-Rogacz M, Chakraborty D, Beschorner N, Hofmann-Sieber H, Lange UC, Grundhoff A, Hackmann K, Schrock E, Abi-Ghanem J, Pisabarro MT, Surendranath V, Schambach A, Lindner C, van Lunzen J, Hauber J, Buchholz F. Directed evolution of a recombinase that excises the provirus of most HIV-1 primary isolates with high specificity. Nat Biotechnol. 2016 Apr;34(4):401-9.

### CRISPR-Cas:

Modzelewski AJ, Chen S, Willis BJ, Lloyd KCK, Wood JA, He L. Efficient mouse genome engineering by CRISPR-EZ technology. Nat Protoc. 2018 Jun;13(6):1253-1274.

### Antikörper:

Antikörper gegen Zytokine wie TNFa oder II-1b zur Behandlung von Autoimmunerkrankungen: Susan J. Lee, MD,a,b Javier Chinen, MD, PhD,c and Arthur Kavanaugh, M Immunomodulator therapy: Monoclonal antibodies, fusion proteins, cytokines, and immunoglobulins. J Allergy Clin Immunol. 2010, 125 (2).

Chimeric Antigen Receptor T cell therapy: Cartellieri M, Feldmann A, Koristka S, Arndt C, Loff S, Ehninger A, von Bonin M, Bejestani EP, Ehninger G, Bachmann MP. Switching CAR T cells on and off: a novel modular platform for retargeting of T cells to AML blasts. Blood Cancer J. 2016 Aug 12;6(8):e458.

Antikörper gegen PD-1 in der Immuntherapie von verschiedenen Krebsarten: Riley JL. Combination checkpoint blockade--taking melanoma immunotherapy to the next level. N Engl J Med. 2013 Jul 11;369(2):187-9. doi: 10.1056/NEJMe1305484. Epub 2013 Jun 2.

Antikörper gegen TGFbeta in der Therapie der Anämie bei MDS: Platzbecker et al. Lancet Oncology 2017.

In Ausführungsformen der Erfindung ist der Inhibitor ein Antikörper oder Fragment davon. Unter einem Fragment wird im Sinne der vorliegenden Erfindung ein Antikörper-Fragment verstanden, welches zumindest eine Tfr2 bindende Sequenz umfasst. Fragmente können dabei bespielhaft scFv-, Fab- oder Fc-Fragmente sein.

In Ausführungsformen der Erfindung ist der Inhibitor ein bispezifische Antikörper. Dabei weist der bispezifische Antikörper eine erste Affinität für Tfr2 auf und eine zweite Affinität für ein weiteres gewebespezifisches Membranprotein. In Ausführungsformen der Erfindung sind die gewebespezifischen Membranproteine Glykoproteine. Bevorzugt sind die Membranproteine CD-Antigene (Cluster of Differentiation).

Der Begriff Cluster of Differentiation ("Unterscheidungsgruppen"), abgekürzt CD, bezeichnet Gruppen immunphänotypischer Oberflächenmerkmale von Zellen, die sich nach biochemischen oder funktionellen Kriterien ordnen lassen. Aufgrund ihrer gewebsspezifischen Expression lassen sich die CD-Moleküle zum gewebsspezifischen Targeting des bispezifischen Antikörpers nutzen. Dadurch kann der Tfr2 gewebsspezifisch inhibiert werden.

Gegenstand der Erfindung ist auch eine pharmazeutische Zusammensetzung umfassend mindestens einen erfindungsgemäßen Inhibitor des Transferrinrezeptor-2.

In Ausführungsformen der Erfindung ist die pharmazeutische Zusammensetzung eine Lösung, Tablette oder Kapsel. In Ausführungsformen der Erfindung wird der erfindungsgemäße Inhibitor als Beschichtung für Implantatmaterialien, bevorzugt Metalle oder Kunststoffe; und/oder Implantate, bevorzugt Prothesen, Schrauben oder Nägel oder ähnliches verwendet.

In einer Ausführungsform wird die pharmazeutische Zusammensetzung lokal intraartikulär, intramuskulär oder systemisch subkutan, intravenös oder über eine orale Gabe verabreicht. In einer Ausführungsform ist die pharmazeutische Zusammensetzung in einer passenden Form für die intraartikuläre, intramuskuläre, subkutane, intravenöse, inhalativ oder orale Verabreichung.

In einer Ausführungsform, enthält die pharmazeutische Zusammensetzung den erfindungsgemäßen Inhibitor in einer Dosis von 10 µg/kg bis 100 mg/kg Körpergewicht pro Verabreichung.

In einer weiteren Ausführungsform enthält die pharmazeutische Zusammensetzung weiterhin ein pharmazeutisch akzeptables Verdünnungsmittel oder Trägermaterial. In einer Ausführungsform ist das pharmazeutisch akzeptable Verdünnungsmittel oder Trägermaterial eine wässrige Lösung, bevorzugt eine gepufferte wässrige Lösung, eine wässrige Salzlösung oder wässrige Glycinlösung. In einer Ausführungsform ist die gepufferte wässrige Lösung ausgewählt aus einer Histidin-gepufferten wässrigen Lösung mit einem pH-Wert von pH 5,0 bis pH 7,0, oder einer Natriumsuccinat-, Natriumcitrat-, Natriumphosphat-, oder Kaliumphosphat-gepufferten wässrigen Lösung. In einer Ausführungsform hat die gepufferte wässrige Lösung eine Konzentration von 1 mmol/l (mM) bis 500 mM, bevorzugt 1 mM bis 50 mM. In einer weiteren Ausführungsform, umfasst das pharmazeutisch akzeptable Verdünnungsmittel oder Trägermaterial Natriumchlorid, bevorzugt in einer Konzentration zwischen 0 mM und 300 mM, besonders bevorzugt in einer Konzentration von 150 mM.

Erfindungsgemäß umfasst die pharmazeutische Zusammensetzung weiterhin mindestens einen pharmazeutisch akzeptablen Hilfsstoff. Unter einem "Hilfsstoff" wird eine Verbindung verstanden, welche physiologische Bedingungen, hinsichtlich des pH-Werts und/oder der lonenstärke, einstellt und/oder die Stabilität der pharmazeutischen Zusammensetzung erhöht. In einer Ausführungsform ist mindesten ein pharmazeutisch akzeptabler Hilfsstoff ausgewählt aus Natriumacetat, Natriumchlorid, Kaliumchlorid, Calciumchlorid oder Natriumlactat.

In Ausführungsformen der Erfindung ist die pharmazeutische Zusammensetzung steril. Die pharmazeutische Zusammensetzung wird durch bekannte Methoden sterilisiert.

Ausführungsformen der Erfindung betreffen einen Inhibitor zur Verwendung bei der Behandlung von primären und sekundären Osteoporosen.

Neue Daten aus unserem Labor zeigen, dass Tfr2 auch im Knochen exprimiert wird. Tfr2-defiziente Mäuse weisen eine zweifach erhöhte Knochenmasse auf, wobei sowohl die Knochenformation als auch Knochenresorption gesteigert ist. Diese Daten legen nahe, dass die Blockade von Tfr2 sowohl positive Effekte auf die Erythropoese als auch auf den Knochenstoffwechsel ausüben könnte

Ebenfalls offenbart, aber nicht gemäß der beanspruchten Erfindung, ist ein Inhibitor zur Verwendung bei der Behandlung der tumor-bedingten Anämie bzw. der Anämie im Rahmen chronischer Erkrankungen (Entzündung, Dialyse, Diabetes etc.). In dieser Situation kommt es einer Hochregulierung von Hepcidin, welches via Degradation von Ferroportin zu einer Eisenretention in den Monozyten und Makrophagen führt. Eine lösliche Form des TFR2 würde als Ligandenfalle (BMPs) agieren und damit gleichsam die TFR2 Signalkaskade hemmen, was zu einer Herunterregulierung der Expression von Hepcidin in der Leber und dann zu einer vermehrten Eisenresorption bzw. zur besseren Bereitstellung aus dem RHS führt.

Ebenfalls offenbart, aber nicht gemäß der beanspruchten Erfindung, ist ein Inhibitor zur Verwendung bei der Behandlung von myelodysplastischen Syndromen, beta-Thalassämie, Niereninsuffizienz, und der Anämie bei chronischer Erkrankung (anemia of chronic disease; ACD).

Für die Realisierung der Erfindung ist es auch zweckmäßig, die vorbeschriebenen Ausführungsformen und Merkmale der Ansprüche zu kombinieren.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

### Ausführungsbeispiele

### Es zeigen die

- Fig. 1: ein Diagramm zum unterschiedlichen trabekulären Knochenvolumen,
- Fig. 2: ein Diagramm zur unterschiedlichen Dicke der Kortikalis,
- Fig. 3: ein Diagramm zur unterschiedlichen Knochenqualität,
- Fig. 4: ein Diagramm zur unterschiedlichen Knochenformation und
- Fig. 5: ein Diagramm zur unterschiedlichen Knochenresorption.

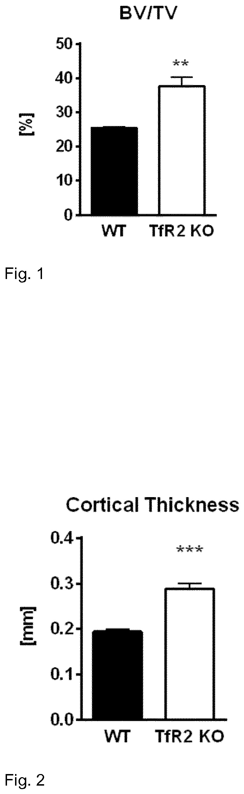

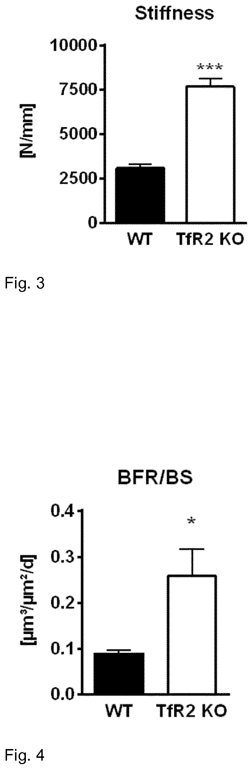

In einem Ausführungsbeispiel zeigen die Knochenmessungen mittels Mikro-Computertomographie von Tfr2-defizienten Mäusen, dass der Verlust von Tfr2 zu einem höheren trabekulären Knochenvolumen (BV/TV) (Fig.1) und einer dickeren Kortikalis in Mäusen führt (Fig. 2). Zudem ist die Qualität des Knochens in der Abwesenheit von Tfr2 besser (Stiffness-Parameter) (Fig. 3) als im Vergleich zu Wildtyp-Mäusen (WT). Die mechanischen Eigenschaften wie die Bruchfestigkeit des Knochens wurde mittels eines Drei-Punkt-Biegetest bestimmt, bei dem die Kraft ermittelt wird, die aufgebracht werden muss um den Knochen zu brechen.

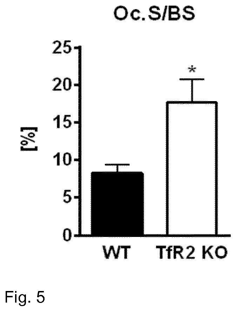

Die Analysen von Tfr2-defizienten Mäusen zeigen ferner in Fig. 4 und Fig. 5, dass die Deletion von Tfr2 zu einem höheren Knochenumsatz führt, also zu einer höheren Knochenformation (bone formation rate per bone surface (BFR/BS) (Fig. 4) und zu einer höheren Knochenresorption (osteoclast surface per bone surface (Oc.S/BS) (Fig. 5). Diese wurden anhand histologischer Untersuchungen ermittelt.

## Patentansprüche

1. Inhibitor des Transferrinrezeptor-2 zur Verwendung bei der Behandlung von Osteoporose, wobei der Inhibitor ausgewählt ist aus einem Antikörper oder Fragment davon, RNA-Interferenz, CRISPR-Cas und einer Rekombinase.

2. Inhibitor zur Verwendung nach Anspruch 1, wobei der Inhibitor ausgewählt ist aus der Gruppe bestehend aus siRNA, shRNA, miRNA, und CRISPR-Cas.

3. Inhibitor zur Verwendung nach Anspruch 1, wobei der Inhibitor ein Antikörper oder Fragment davon ist.

4. Pharmazeutische Zusammensetzung zur in Anspruch 1 genannten Verwendung umfassend zumindest einen Inhibitor nach einem der Ansprüche 1 bis 3 sowie einen pharmazeutisch akzeptablen Hilfsstoff.

## Claims

1. Inhibitor of transferrin receptor 2 for use in the treatment of osteoporosis, wherein the inhibitor is selected from an antibody or fragment thereof, RNA interference, CRISPR-Cas and a recombinase.

2. Inhibitor for use according to claim 1, wherein the inhibitor is selected from the group consisting of siRNA, shRNA, miRNA and CRISPR-Cas.

3. Inhibitor for use according to claim 1, wherein the inhibitor is an antibody or fragment thereof.

4. Pharmaceutical composition for the use stated in claim 1, comprising at least one inhibitor according to any of claims 1 to 3 and also a pharmaceutically acceptable excipient.

## Revendications

1. Inhibiteur du récepteur 2 de la transferrine pour une utilisation dans le traitement de l'ostéoporose, l'inhibiteur étant choisi parmi un anticorps et un fragment de celui-ci, une interférence par ARN, un CRISPR-Cas et une recombinase.

2. Inhibiteur pour une utilisation selon la revendication 1, l'inhibiteur étant choisi dans le groupe constitué par un ARNsi, un ARNsh, un ARNmi et un CRISPR-Cas.

3. Inhibiteur pour une utilisation selon la revendication 1, l'inhibiteur étant un anticorps ou un fragment de celui-ci.

4. Composition pharmaceutique pour une utilisation mentionnée dans la revendication 1 comprenant au moins un inhibiteur selon l'une quelconque des revendications 1 à 3 ainsi qu'un excipient pharmaceutiquement acceptable.
